# EUROPEAN PATENT APPLICATION

(11) **EP 4 647 003 A1**
(43) Date of publication of application: **12.11.2025**
(21) Application number: 24769710.5
(22) Date of filing: 18.02.2024
(51) Int. Cl.: A61B 5/022

(54) **AIRBAG, BLOOD PRESSURE MEASUREMENT APPARATUS, AND WEARABLE DEVICE**

(30) Priority: 10.03.2023 CN 202320533614 U
(71) Applicant: Huawei Technologies Co., Ltd., Shenzhen, Guangdong 518129 (CN)
(72) Inventor: TIAN, Guoqiang, Shenzhen, Guangdong 518129 (CN); CHENG, Tianyu, Shenzhen, Guangdong 518129 (CN); WANG, Youhua, Shenzhen, Guangdong 518129 (CN); JIN, Junye, Shenzhen, Guangdong 518129 (CN); XIE, Yun, Shenzhen, Guangdong 518129 (CN); YUAN, Shenglan, Shenzhen, Guangdong 518129 (CN); LI, Changming, Shenzhen, Guangdong 518129 (CN); LIN, Yichao, Shenzhen, Guangdong 518129 (CN)
(74) Representative: Maiwald GmbH
(86) International application number: PCT/CN2024/077405
(87) International publication number: WO 2024/188015

(57) **Abstract**

Embodiments of this application provide an airbag, a blood pressure measurement apparatus, and a wearable device, to resolve a problem of poor wearing comfort of the blood pressure measurement apparatus in a related technology. The airbag includes a first film layer and a second film layer that are disposed opposite to each other. The first film layer is configured to be in contact with skin. The first film layer includes a first body part and a first edge part, and the first edge part is adjacent to the first body part and is disposed along a circumferential direction of the first body part. The second film layer includes a second body part and a second edge part, and the second edge part is adjacent to the second body part and is disposed along a circumferential direction of the second body part. The first body part and the second body part enclose a first cavity, and the first edge part and the second edge part that are connected and disposed in a stacked manner jointly form a first connection part. The first connection part is located in the first cavity. During actual use, contact friction between at least a portion of the connection part and skin of a wrist can be avoided, and irritation to the skin of the wrist can be reduced, thereby improving wearing comfort of a user.

## Description

This application claims priority to Chinese Patent Application No. 202320533614.0, filed with the China National Intellectual Property Administration on March 10, 2023 and entitled "AIRBAG, BLOOD PRESSURE MEASUREMENT APPARATUS, AND WEARABLE DEVICE", which is incorporated herein by reference in its entirety.

### TECHNICAL FIELD

Embodiments of this application relate to the field of blood pressure measurement technologies, and in particular, to an airbag, a blood pressure measurement apparatus, and a wearable device.

### BACKGROUND

With continuous improvement of living standards, people pay more attention to their own health. Blood pressure, as a main physiological indicator of a human body, is also increasingly valued by people. Currently, blood pressure monitor products available on markets include blood pressure watches, which are compact and wearable blood pressure measurement apparatuses. A blood pressure watch usually includes a watch core, a watch strap, an airbag, an air pump, and a barometric pressure sensor. Both the airbag and the watch strap may be sleeved on a wrist. Both the air pump and the barometric pressure sensor are disposed in the watch core. The air pump is configured to inflate the airbag. The barometric pressure sensor is configured to collect a pressure signal in the airbag to obtain a pulse wave signal, to implement blood pressure measurement. However, blood pressure watches in a related technology further have a problem of poor wearing comfort.

### SUMMARY

An objective of embodiments of this application is to provide an airbag, a blood pressure measurement apparatus, and a wearable device, to resolve a problem of poor wearing comfort of the blood pressure measurement apparatus in a related technology.

To achieve the foregoing objective, embodiments of this application provide the following solutions.

In one aspect, an airbag for blood pressure measurement is provided, where the airbag includes a first film layer and a second film layer. The first film layer and the second film layer are disposed opposite to each other. The first film layer is configured to be in contact with skin. The first film layer includes a first body part and a first edge part, the first edge part is adjacent to the first body part, and the first edge part is disposed along a circumferential direction of the first body part. The second film layer includes a second body part and a second edge part, the second edge part is adjacent to the second body part, and the second edge part is disposed along a circumferential direction of the second body part. The first body part and the second body part enclose a first cavity, the first edge part and the second edge part are connected and disposed in a stacked manner, and the first edge part and the second edge part jointly form a first connection part. According to the foregoing configuration, the first edge part and the second edge part may form the first connection part by using a pressing process. The first film layer and the second film layer may be connected through the first edge part that is pressed together, to facilitate the first body part and the second body part to enclose a sealing cavity, and the sealing cavity is the first cavity.

According to the foregoing configuration, the first connection part is located in the first cavity. During actual use, because the first connection part is located in the first cavity, contact friction between the first connection part and skin of a wrist can be avoided, and irritation to the skin of the wrist can be reduced, thereby improving wearing comfort of a user.

In addition, because the first connection part is located in the first cavity, areas of the first body part and the second body part can be increased on a basis of an unchanged overall size of the airbag, so that a volume of the first cavity enclosed by the first body part and the second body part can be increased. This helps increase an inflation amount of the airbag and improve measurement accuracy of the blood pressure measurement apparatus. On the contrary, according to the foregoing configuration, the size of the airbag can be reduced on a basis of same measurement accuracy of the blood pressure measurement apparatus. For example, when the airbag has a same width, compared with an effective inflation area in embodiments of the related technology, an effective inflation area in embodiments of this application is increased by twice a width of the first connection part.

In some embodiments, the first edge part and the second edge part further jointly form a second connection part, the second connection part and the first connection part are connected end-to-end in a ring shape and jointly form a connection part, and the second connection part is located outside the first cavity. The first connection part and the second connection part are disposed, so that the first film layer and the second film layer are pressed together to form the first cavity.

In some embodiments, the connection part includes a first portion, a second portion, a third portion, and a fourth portion, the first portion and the second portion are disposed opposite to each other, the third portion and the fourth portion are disposed opposite to each other, and the third portion and the fourth portion are connected between the first portion and the second portion. The first connection part includes the first portion, the second portion, and the third portion, and the second connection part includes the fourth portion. According to the foregoing configuration, a length of the first connection part can be further increased, so that contact friction between the first connection part and the skin of the wrist can be avoided, and irritation to the skin of the wrist can be reduced, thereby improving wearing comfort of the user.

In some embodiments, the airbag includes a third film layer and a fourth film layer, and the third film layer and the fourth film layer are disposed opposite to each other. The third film layer includes a third body part and a third edge part, the third edge part is adjacent to the third body part, and the third edge part is disposed along a circumferential direction of the third body part. The fourth film layer includes a fourth body part and a fourth edge part, the fourth edge part is adjacent to the fourth body part, and the fourth edge part is disposed along a circumferential direction of the fourth body part. The third body part and the fourth body part enclose a second cavity, the third edge part and the fourth edge part are connected and disposed in a stacked manner, and the third body part is connected to the second body part. The third body part includes a second opening, the second body part includes a first opening, and the second opening is in communication with the first opening. The second cavity is disposed, so that the airbag can be disposed in a double-layer manner. On a premise of an unchanged overall inflation volume of the airbag, a width of the airbag can be narrowed, so that a width of a watch strap on which the airbag is mounted is narrowed, thereby improving wearing comfort of the blood pressure measurement apparatus.

In some embodiments, the airbag further includes an air nozzle, and the air nozzle is in communication with the second cavity and is connected between the third edge part and the fourth edge part. According to the foregoing configuration, the air pump can inflate the second cavity through the air nozzle, and air in the second cavity enters the first cavity, so that the first film layer of the first cavity presses radial artery blood vessels and ulnar artery blood vessels of a wrist of a human body, thereby facilitating blood pressure measurement.

In some embodiments, a first connection film is further included. The first opening includes a first edge and a second edge that are disposed opposite to each other, the first connection film is connected between the first edge and the second edge, and a plurality of the first connection films are spaced apart. In an expansion process of the first cavity, the plurality of the first connection films are disposed, so that a limiting effect can be achieved on the first edge and the second edge of the first opening. This helps reduce a deformation speed of the second film layer in which the first opening is located, thereby improving stability of the first cavity and measurement accuracy of the blood pressure measurement apparatus.

In some embodiments, the first connection film includes a bent part, and/or the first connection film includes an arc-shaped part. In the expansion process of the first cavity, the bent part and/or the arc-shaped part are disposed in the first connection film, so that the first connection film can have a specific stretching amount (herein, the stretching amount is a length that the first connection film can stretch in a direction perpendicular to the first edge), so that the second body part in which the first opening is located has a specific deformation amount. This helps increase an expansion height and an inflation volume of the first cavity enclosed by the second body part and the first body part, thereby improving measurement accuracy of the blood pressure measurement apparatus.

In some embodiments, a second connection film is further included. The second opening includes a third edge and a fourth edge that are disposed opposite to each other, the second connection film is connected between the third edge and the fourth edge, a plurality of the second connection films are spaced apart, and the plurality of the second connection films are disposed symmetric to the plurality of the first connection films. The plurality of the second connection films are disposed symmetric to the plurality of the first connection films, so that a communication area between the first opening and the second opening can be increased, and a ventilation volume between the first cavity and the second cavity can be increased. This can increase the expansion height and the inflation volume of the first cavity, thereby improving measurement accuracy of the blood pressure measurement apparatus.

In some embodiments, the airbag further includes a first intermediate film layer and a second intermediate film layer, the first intermediate film layer and the second intermediate film layer enclose an intermediate cavity, and the intermediate cavity is located between the first cavity and the second cavity. The airbag further includes a bonding part, the bonding part is configured to connect the second film layer, the first intermediate film layer, the second intermediate film layer, and the third film layer, the bonding part is located between the first cavity and the second cavity, the intermediate cavity is disposed along a circumferential direction of the bonding part, and the second cavity is in communication with the first cavity through the intermediate cavity. According to the foregoing configuration, in an inflation process of the airbag, because a volume of the intermediate cavity expands, the intermediate cavity squeezes the second film layer in contact with the intermediate cavity, so that an edge position of the second body part of the second film layer moves toward a direction close to the wrist. This increases an effective pressing area between the first film layer and the wrist, thereby improving measurement accuracy of the blood pressure measurement apparatus. Herein, the effective pressing area is an area in which the first film layer can press the radial artery blood vessels and the ulnar artery blood vessels of the wrist of the human body to enable the blood pressure measurement apparatus to obtain the pulse wave signal.

In addition, because the intermediate cavity is disposed in the circumferential direction of the bonding part, the intermediate cavity can stabilize the first cavity. This helps prevent the first cavity from deviating toward one side, thereby improving stability of the first cavity and measurement accuracy of the blood pressure measurement apparatus.

In some embodiments, the first intermediate film layer includes a first intermediate opening, the first intermediate opening is in communication with the first opening, the second intermediate film layer includes a second intermediate opening, and the second intermediate opening is in communication with the second opening. According to the foregoing configuration, the first cavity can be in communication with the second cavity through the intermediate cavity.

In some embodiments, the second film layer, the first intermediate film layer, the second intermediate film layer, and the third film layer are sequentially stacked to form the bonding part. According to the foregoing configuration, connection strength between the second film layer, the first intermediate film layer, the second intermediate film layer, and the third film layer can be improved.

In another aspect, a blood pressure measurement apparatus is provided, including a mounting member, a pipeline, an air pump, and the airbag in any one of the foregoing embodiments. Both the pipeline and the airbag are located in the mounting member. One end of the pipeline is in communication with the air pump, and the other end of the pipeline is in communication with the airbag. The blood pressure measurement apparatus provided in embodiments of this application includes the foregoing airbag, and therefore has all the foregoing beneficial effects. Details are not described herein again.

In some embodiments, the pipeline includes a first pipe section, a telescopic member, and a second pipe section, the telescopic member is in communication with both the first pipe section and the second pipe section, the first pipe section is connected to the mounting member, and the second pipe section is in communication with the airbag. According to the foregoing configuration, when a bellow is compressed to an end of a watch head, the pipeline can drive the airbag to move toward an end close to the watch head. When the bellow is stretched to an end of the airbag, the pipeline can drive the airbag to move toward an end away from the watch head. According to a telescopic structure of the pipeline, a position of the airbag can be correspondingly adjusted for different users, so that the airbag can cover the radial artery blood vessels and the ulnar artery blood vessels of the wrist, thereby improving measurement accuracy of the blood pressure measurement apparatus. In other words, on a premise of an unchanged size of the airbag, a range of the wrist that can be covered by the airbag increases, thereby increasing a measurement range of the blood pressure measurement apparatus.

In some embodiments, the telescopic member includes the bellow. The bellow has a simple structure, and can help improve reliability of blood pressure measurement apparatus.

In another aspect, a wearable device is provided. The wearable device provided in embodiments of this application includes the foregoing blood pressure measurement apparatus, and therefore has all the foregoing beneficial effects. Details are not described herein again.

In some embodiments, an inflation part, a first valve body, and a second valve body are further included. The inflation part includes an inflation cavity, an air inlet hole of the inflation cavity is in communication with external air, the first valve body is located in the air inlet hole of the inflation cavity, the first valve body is configured to block or open the air inlet hole of the inflation cavity, an air outlet hole of the inflation cavity is in communication with an airbag, the second valve body is located in the air outlet hole of the inflation cavity, and the second valve body is configured to block or open the air outlet hole of the inflation cavity. The airbag can be manually inflated by manually and repeatedly pressing the inflation part, so that a volume of the airbag can be increased. This can reduce a gap between the watch strap and the wrist, thereby facilitating attachment of a blood pressure watch to the wrist.

In some embodiments, an air release part and a third valve body are further included. The air release part includes an air release cavity, an air inlet of the air release cavity is in communication with the airbag, an air outlet of the air release cavity is in communication with the external air, the third valve body is located in the air release cavity, the third valve body is configured to block or open the air outlet of the air release cavity, and the third valve body is further configured to block or open the air inlet of the air release cavity. When the user needs to relax the wrist, the user may manually press the third valve body to release air from the airbag, so that the volume of the airbag is reduced. This increases the gap between the watch strap and the wrist, thereby improving wearing experience.

### BRIEF DESCRIPTION OF DRAWINGS

FIG. 1 is a diagram of a structure of a wearable device according to an embodiment of this application;
FIG. 2 is a diagram of a structure of another wearable device according to an embodiment of this application;
FIG. 3 is a cross-sectional view of a watch strap of another blood pressure measurement apparatus according to an embodiment of this application;
FIG. 4 is a block diagram of a blood pressure measurement apparatus according to an embodiment of this application;
FIG. 5 is a diagram of a structure of a telescopic member that is of a watch strap and that is in a compressed state according to an embodiment of this application;
FIG. 6 is a cross-sectional view of the watch strap in FIG. 5;
FIG. 7 is a diagram of a structure of the telescopic member in FIG. 5;
FIG. 8 is a diagram of a structure of a telescopic member that is of a watch strap and that is in a stretched state according to an embodiment of this application;
FIG. 9 is a diagram of a structure of the telescopic member in FIG. 8;
FIG. 10 is a diagram of a structure of an airbag according to an embodiment of this application;
FIG. 11 is a cross-sectional view of the airbag in FIG. 10 along a section line C-C;
FIG. 12 is a diagram of a structure of a first film material and a second film material according to an embodiment of this application;
FIG. 13 is a diagram of a structure of a first intermediate body according to an embodiment of this application;
FIG. 14 is a diagram of a structure of a first intermediate body that is flipped according to an embodiment of this application;
FIG. 15 is a diagram of a structure of a formed second connection part according to an embodiment of this application;
FIG. 16 is a diagram of a structure of another airbag according to an embodiment of this application;
FIG. 17 is a cross-sectional view of the airbag in FIG. 16 along a section line D-D;
FIG. 18 is a diagram of a structure of a third film material and a fourth film material according to an embodiment of this application;
FIG. 19 is a diagram of a structure of a second intermediate body according to an embodiment of this application;
FIG. 20 is a diagram of a structure of an air nozzle according to an embodiment of this application;
FIG. 21 is a diagram of a structure of another air nozzle according to an embodiment of this application;
FIG. 22 is a diagram of a structure of a second film layer according to an embodiment of this application;
FIG. 23 is a diagram of a structure of another second film layer according to an embodiment of this application;
FIG. 24 is a diagram of a structure of another second film layer according to an embodiment of this application;
FIG. 25 is a diagram of a structure of another second film layer according to an embodiment of this application;
FIG. 26 is a diagram of a structure of another second film layer according to an embodiment of this application;
FIG. 27 is a diagram of a structure of another second film layer according to an embodiment of this application;
FIG. 28 is a diagram of structures of a second film layer and a third film layer according to an embodiment of this application;
FIG. 29 is a diagram of a structure of another airbag according to an embodiment of this application;
FIG. 30 is a diagram of a structure of a mold according to an embodiment of this application;
FIG. 31 is a diagram of a structure of another airbag according to an embodiment of this application;
FIG. 32 is a cross-sectional view of the airbag in FIG. 31 along a section line E-E;
FIG. 33 is a diagram of a structure of another airbag according to an embodiment of this application; and
FIG. 34 is a cross-sectional view of the airbag in FIG. 33 along a section line F-F.

### DESCRIPTION OF EMBODIMENTS

The following describes technical solutions in embodiments of this application with reference to accompanying drawings in embodiments of this application. It is clear that the described embodiments are merely a part rather than all of embodiments of this application.

Terms such as "first" and "second" below are merely for convenience of description, and are not to be construed as indicating or implying relative importance or implicitly indicating a quantity of indicated technical features. Therefore, a feature limited by "first", "second", or the like may explicitly or implicitly include one or more features. In the descriptions of this application, unless otherwise stated, "a plurality of" means two or more than two.

In addition, in embodiments of this application, the word "exemplary" or "for example" is used to represent giving an example, an illustration, or a description. Any embodiment or design scheme described as an "example" or "for example" in embodiments of this application should not be explained as being more preferred or having more advantages than another embodiment or design scheme. Exactly, use of the terms such as "example" or "for example" is intended to present a related concept in a specific manner.

In embodiments of this application, "and/or" describes an association relationship between associated objects, and represents that three relationships may exist. For example, A and/or B may represent the following cases: Only A exists, both A and B exist, and only B exists, where A and B may be singular or plural. The character "/" generally indicates an "or" relationship between the associated objects.

In embodiments of this application, for example, up, down, left, right, front, and rear are relative direction indications used to explain structures and movement of different parts in this application. These indications are appropriate when the parts are in positions shown in the figure. However, if descriptions of the positions of the parts change, these direction indications correspondingly change.

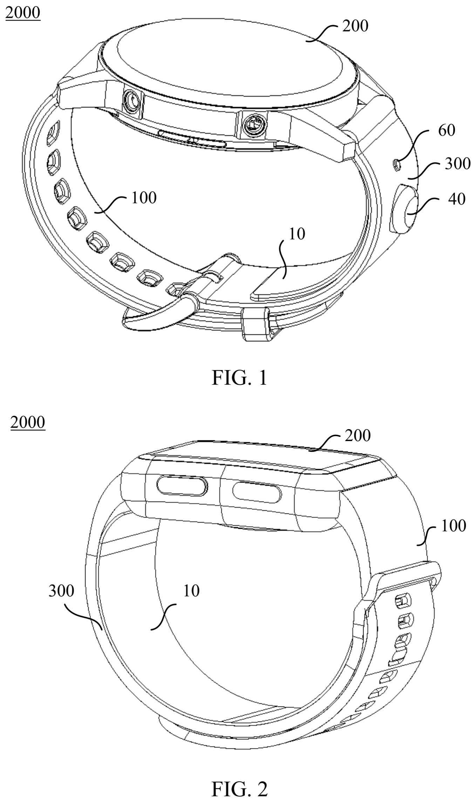

FIG. 1 is a diagram of a structure of a wearable device 2000 according to an embodiment of this application. FIG. 2 is a diagram of a structure of another wearable device 2000 according to an embodiment of this application. As shown in FIG. 1 and FIG. 2, the wearable device 2000 provided in embodiments of this application may be an electronic device such as a sports band or a blood pressure watch.

Still refer to FIG. 1 and FIG. 2. The wearable device 2000 may include a watch head 200 and watch straps 100, and the watch head 200 is connected to the watch strap 100. The watch straps 100 are respectively disposed on two opposite sides of the watch head 200. A watch buckle may be disposed on a watch strap 100 located on a side of the watch head 200, and a buckle hole may be provided on a watch strap 100 located on the other side of the watch head 200. During use, the watch straps 100 located on the two sides of the watch head 200 may be sleeved on a wrist of a human body through cooperation between the watch buckle and the buckle hole.

The wearable device 2000 may include a blood pressure measurement apparatus 1000. The blood pressure measurement apparatus 1000 may include a mounting member 300 and an airbag 10. The mounting member 300 may be, for example, located in the watch strap 100. The airbag 10 may be located in the mounting member 300, so that the airbag 10 can be attached to the human body, thereby facilitating use of the airbag 10 for blood pressure measurement.

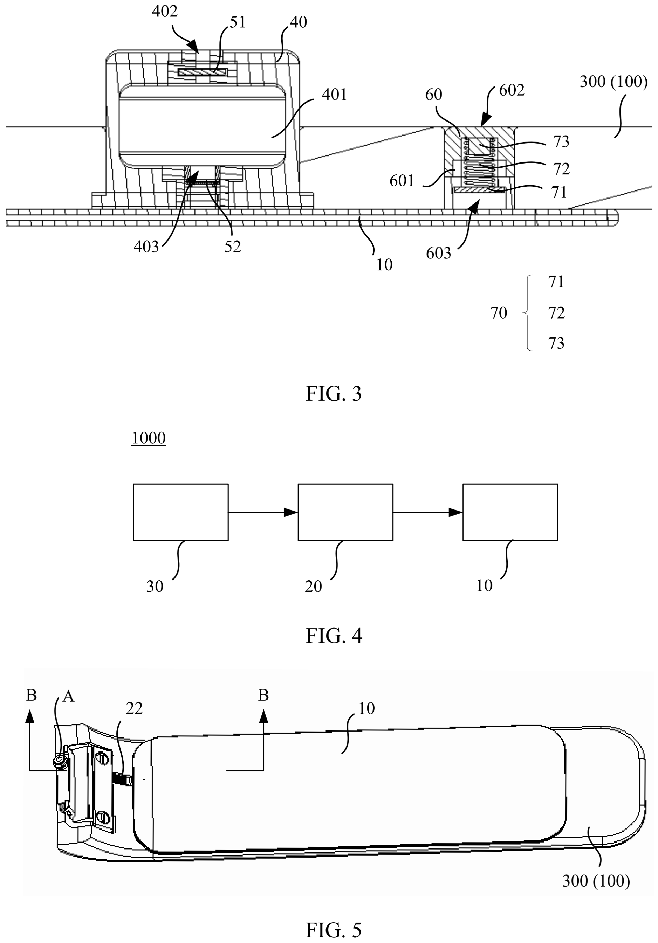

FIG. 3 is a cross-sectional view of a watch strap of another wearable device 2000 according to an embodiment of this application.

As shown in FIG. 3, in some embodiments, the wearable device 2000 may further include an inflation part 40. The inflation part 40 includes an inflation cavity 401. An air inlet hole 402 of the inflation cavity 401 is in communication with external air, and an air outlet hole 403 of the inflation cavity 401 is in communication with the airbag 10. For example, the inflation part 40 may be located on a side that is of the mounting member 300 and that is away from the wrist. An end of the inflation part 40 extends into the mounting member 300 and is connected to the airbag 10, and the other end of the inflation part 40 is exposed outside the mounting member 300. For example, the inflation part 40 may be approximately a hollow cylinder. Correspondingly, the inflation cavity 401 may also be approximately a cylinder. An end that is of the inflation part 40 and that is away from the airbag 10 has a through hole, and the through hole is in communication with both the external air and the inflation cavity 401. The through hole is the air inlet hole 402 of the inflation cavity 401. The external air may enter the inflation cavity 401 through the air inlet hole 402 of the inflation cavity 401. The other end that is of the inflation part 40 and that is close to the airbag 10 also has a through hole, and the through hole is in communication with both the inflation cavity 401 and the airbag 10. The through hole is the air outlet hole 403 of the inflation cavity 401, and air in the inflation cavity 401 may enter the airbag 10 through the air outlet hole 403 of the inflation cavity 401.

Based on the foregoing structure, as shown in FIG. 3, the wearable device 2000 may further include a first valve body 51 and a second valve body 52. The first valve body 51 is located in the air inlet hole 402 of the inflation cavity 401, and the first valve body 51 is configured to block or open the air inlet hole 402 of the inflation cavity 401. The second valve body 52 is located in the air outlet hole 403 of the inflation cavity 401, and the second valve body 52 is configured to block or open the air outlet hole 403 of the inflation cavity 401. For example, shapes of the first valve body 51 and the second valve body 52 may be respectively configured based on shapes of the air inlet hole 402 and the air outlet hole of the inflation cavity 401. For example, the first valve body 51 may be of a flat plate structure, and the second valve body 52 may be a valve flap.

According to the foregoing configuration, when the inflation part 40 is pressed, the inflation cavity 401 is squeezed, and the first valve body 51 moves toward an end away from the mounting member 300 under an action of air pressure in the inflation cavity 401, so that the first valve body 51 blocks the air inlet hole 402 of the inflation cavity 401, thereby preventing the air in the inflation cavity 401 from leaking out through the air inlet hole 402 of the inflation cavity 401. In addition, the second valve body 52 moves toward an end close to the mounting member 300 under the action of the air pressure in the inflation cavity 401, so that the second valve body 52 is in communication with the air outlet hole 403 of the inflation cavity 401, and the air in the inflation cavity 401 can be inflated into the airbag 10 through the air outlet hole 403 of the inflation cavity 401.

Further, when the inflation part 40 is released, the inflation cavity 401 is restored to an original volume, and the second valve body 52 moves toward an end away from the airbag 10 under the action of the air pressure in the inflation cavity 401, so that the second valve body 52 blocks the air outlet hole 403 of the inflation cavity 401, thereby preventing air in the airbag 10 from leaking into the inflation cavity 401. In addition, the first valve body 51 moves toward an end close to the airbag 10 under the action of the air pressure in the inflation cavity 401, so that the first valve body 51 is in communication with the air inlet hole 402 of the inflation cavity 401, and the external air can enter the inflation cavity 401 through the air inlet hole 402 of the inflation cavity 401. It can be learned that the airbag 10 can be manually inflated by manually and repeatedly pressing the inflation part 40.

It may be understood that, a user has different requirements on a tightness degree of the watch strap 100 sleeved on the wrist in different use scenarios. For example, when the user wears a blood pressure watch during exercise, a gap between the watch strap 100 and the wrist causes the wearable device 2000 to shake, leading to poor wearing comfort of the wearable device 2000. As described in the foregoing embodiments, the airbag 10 can be manually inflated by manually and repeatedly pressing the inflation part 40, so that a volume of the airbag 10 can be increased. This can reduce a gap between the watch strap 100 and the wrist, thereby facilitating attachment of the wearable device 2000 to the wrist.

In some embodiments, as shown in FIG. 3, the wearable device 2000 may further include an air release part 60 and a third valve body 70. The air release part 60 includes an air release cavity 601. An air inlet 603 of the air release cavity 601 is in communication with the airbag 10, and an air outlet 602 of the air release cavity 601 is in communication with the external air. For example, the air release part 60 is mounted in the mounting member 300, and a side that is of the air release part 60 and that is away from the wrist may be exposed outside the mounting member 300. The air release part 60 may be a hollow cylinder. An end that is of the air release part 60 and that is away from the airbag 10 has an opening that passes through the air release part 60, and the opening is in communication with both the external air and the air release cavity 601. The opening is the air outlet 602 of the air release cavity 601. Air in the air release cavity 601 may enter the external air through the air outlet 602 of the air release cavity 601. An end that is of the air release part 60 and that is close to the airbag 10 has an opening, and the opening is in communication with both the airbag 10 and the air release cavity 601. The opening is the air inlet 603 of the air release cavity 601. The air in the airbag 10 may enter the air release cavity 601 through the air inlet 603 of the air release cavity 601.

Based on the foregoing structure, as shown in FIG. 3, the wearable device 2000 may further include a third valve body 70. The third valve body 70 may be located in the air release cavity 601. The third valve body 70 is configured to block or open the air outlet 602 of the air release cavity 601, and the third valve body 70 is further configured to block or open the air inlet 603 of the air release cavity 601. For example, the third valve body 70 may include a valve flap 71, an elastic body 72, and a positioning block 73. An end that is of the elastic body 72 and that is away from the airbag 10 is connected to the positioning block 73, and an end that is of the elastic body 72 and that is close to the airbag 10 is connected to the valve flap 71.

According to the foregoing configuration, under an action of air pressure in the airbag 10, the valve flap 71 blocks the air inlet 603 of the air release cavity 601. In addition, when the elastic body 72 is in a normal state, the positioning block 73 blocks the air outlet 602 of the air release cavity 601. When the positioning block 73 is pressed by using a tool such as an ejector pin passing through the air outlet 602 of the air release cavity 601, the positioning block 73 moves toward a direction close to the airbag 10, so that the positioning block 73 is in communication with the air outlet 602 of the air release cavity 601. In this case, the elastic body 72 is in a compressed state, and the elastic body 72 drives the valve flap 71 to move toward the direction close to the airbag 10, so that the valve flap 71 is in communication with the air inlet 603 of the air release cavity 601. In other words, in this case, the airbag 10 is in communication with the external air sequentially through the air inlet 603 of the air release cavity 601, the air release cavity 601, and the air outlet 602 of the air release cavity 601, so that the air in the airbag 10 can be released to the external air. It can be learned that the airbag 10 may further implement air release through manual pressing.

As described in the foregoing embodiments, the user has different requirements on the tightness degree of the watch strap 100 sleeved on the wrist in different use scenarios. When the user needs to relax the wrist, the user may manually press the third valve body 70 to release air from the airbag 10, so that the volume of the airbag 10 is reduced. This increases the gap between the watch strap 100 and the wrist, thereby improving wearing experience.

According to the foregoing configuration, it is convenient to wear the wearable device 2000 on a human body to help a blood pressure measurement apparatus 1000 monitor a blood pressure status of the human body in real time.

FIG. 4 is a block diagram of a blood pressure measurement apparatus 1000 according to an embodiment of this application. As shown in FIG. 4, the blood pressure measurement apparatus 1000 further includes a pipeline 20, an air pump 30, and an airbag 10. With reference to FIG. 1 and FIG. 2, it can be learned that both the pipeline 20 and the airbag 10 are located in the mounting member 300, an end of the pipeline 20 is in communication with the air pump 30, and the other end of the pipeline 20 is in communication with the airbag 10. Herein, the airbag 10 may be an airbag 10 in any one of the following embodiments. For example, the airbag 10 and the pipeline 20 are mounted in the watch strap 100, and the air pump 30 is located in a watch head 200. According to the foregoing configuration, the air pump 30 can supply air to the airbag 10 through the pipeline 20, and the airbag 10 inflated with the air presses radial artery blood vessels and ulnar artery blood vessels of a wrist of a human body after the airbag 10 is pressurized and expanded, to extract a pulse wave signal. The blood pressure measurement apparatus 1000 may be worn on the wrist of the human body, and may implement an objective of measuring blood pressure of the human body.

Herein, "blood pressure" is lateral pressure exerted by blood on walls of blood vessels. Both excessively high and low blood pressure can affect health of the human body. "Radial artery" is a lower part that is of a radial artery and that is covered only by skin and fascia, and is a part where a pulse is clinically palpated. The "ulnar artery" is a part that is on an opposite side of the radial artery and that is connected to the radial artery in a palm.

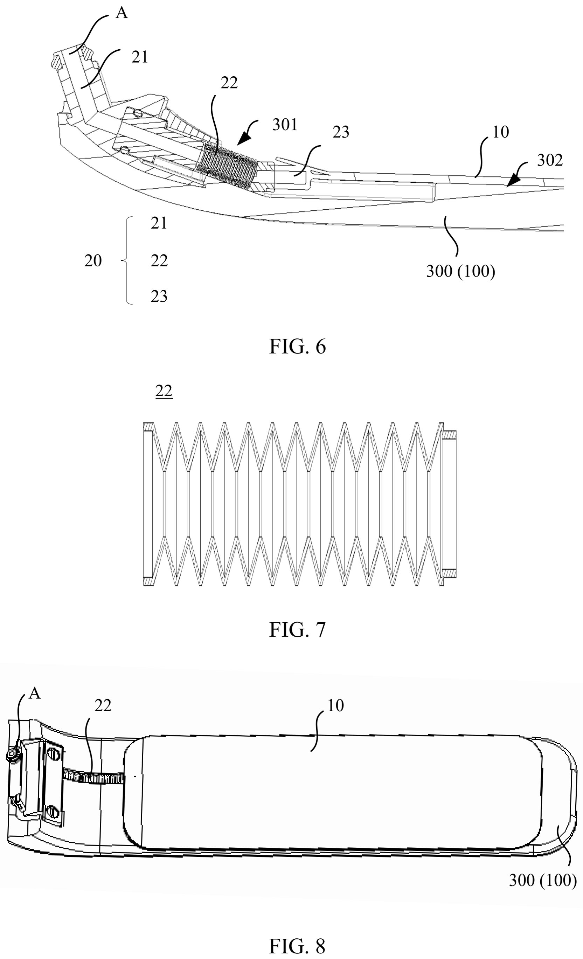

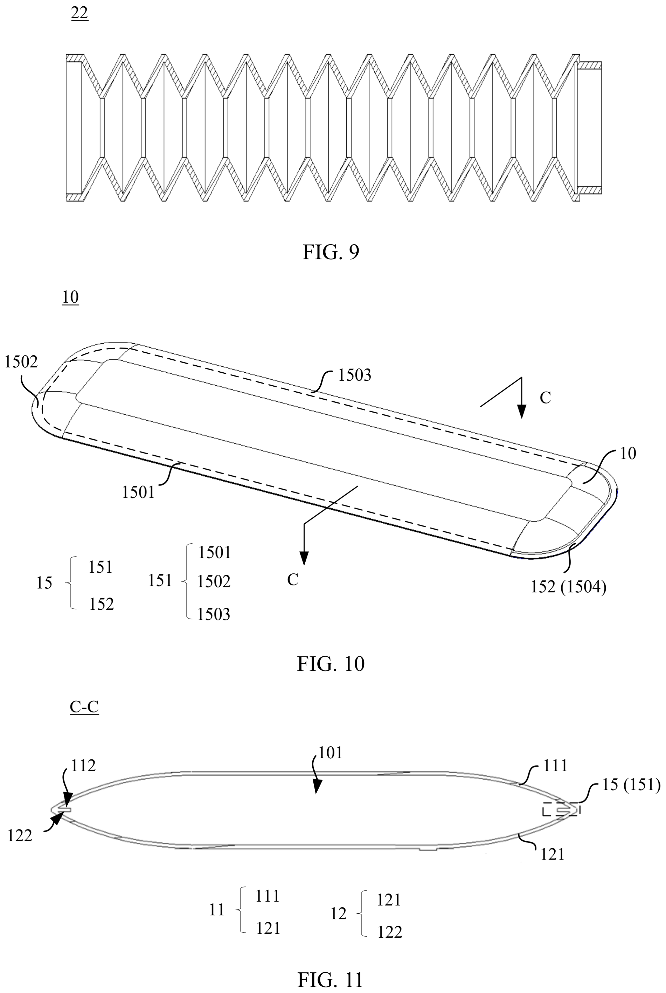

FIG. 5 is a diagram of a structure of a telescopic member 22 that is of a watch strap 100 and that is in a compressed state according to an embodiment of this application. FIG. 6 is a cross-sectional view of the watch strap 100 in FIG. 5. FIG. 7 is a diagram of a structure of the telescopic member 22 in FIG. 5. FIG. 8 is a diagram of a structure of a telescopic member 22 that is of a watch strap and that is in a stretched state according to an embodiment of this application. FIG. 9 is a diagram of a structure of the telescopic member 22 in FIG. 8.

As shown in FIG. 5 and FIG. 6, in some embodiments, the pipeline 20 may include a first pipe section 21, the telescopic member 22, and a second pipe section 23 that are sequentially communicated. The first pipe section 21 is in communication with the mounting member 300, and the second pipe section 23 is in communication with the airbag 10. For example, the first pipe section 21 is located at an end that is of the pipeline 20 and that is close to the watch head 200, and the first pipe section 21 is mounted in the mounting member 300. An end (as shown in A in the figure) that is of the first pipe section 21 and that is away from the telescopic member 22 may be in communication with the air pump 30 in the watch head 200. The mounting member 300 may further include a first mounting groove 301 and a second mounting groove 302. The first mounting groove 301 and the second mounting groove 302 are located on a side that is of the mounting member 300 and that faces the wrist, and the first mounting groove 301 is in communication with the second mounting groove 302. The telescopic member 22 and the second pipe section 23 may be located in the first mounting groove 301, and the airbag 10 may be located in the second mounting groove 302.

It may be understood that, due to differences between personal physiological structures, a position at which the airbag 10 needs to press against the wrist is also different. The telescopic member 22 is disposed, so that the telescopic member 22 drives the airbag 10 to move, thereby adjusting the position at which the airbag 10 presses against the wrist.

In some embodiments, the telescopic member 22 may include a bellow. A material of the pipeline 20 is not limited in embodiments of this application. For example, the material of the pipeline 20 may include one of plastic, rubber, and stainless steel.

As shown in FIG. 5 and FIG. 7, when the bellow is compressed to an end of the watch head 200, the pipeline 20 can drive the airbag 10 to move toward an end close to the watch head 200 (that is, move in a direction close to A). As shown in FIG. 8 and FIG. 9, when the bellow is stretched to an end of the airbag 10, the pipeline 20 can drive the airbag 10 to move toward an end away from the watch head 200 (that is, move in a direction away from A).

In some other embodiments, the telescopic member 22 may alternatively be of another structure that can be telescopic. This is not limited in embodiments of this application.

In conclusion, according to the foregoing configuration, a position of the airbag 10 can be correspondingly adjusted for different users, so that the airbag 10 can cover the radial artery blood vessels and the ulnar artery blood vessels of the wrist, thereby improving measurement accuracy of the blood pressure measurement apparatus 1000. In other words, on a premise of an unchanged size of the airbag 10, a range of the wrist that can be covered by the airbag 10 increases, thereby increasing a measurement range of the blood pressure measurement apparatus 1000.

FIG. 10 is a diagram of a structure of an airbag 10 according to an embodiment of this application. FIG. 11 is a cross-sectional view of the airbag 10 in FIG. 10 along a section line C-C.

As shown in FIG. 10 and FIG. 11, the airbag 10 provided in this embodiment of this application includes a first film layer 11 and a second film layer 12, the first film layer 11 and the second film layer 12 are disposed opposite to each other, and the first film layer 11 is configured to be in contact with skin. For example, shapes of the first film layer 11 and the second film layer 12 may be the same. For example, the shapes of the first film layer 11 and the second film layer 12 may be approximately round rectangles, so that a shape of the airbag 10 is regular. Certainly, in some other embodiments, the shapes of the first film layer 11 and the second film layer 12 may be correspondingly configured based on an actual situation. This is not limited in embodiments of this application.

Still refer to FIG. 11. The first film layer 11 includes a first body part 111 and a first edge part 112, the first edge part 112 is adjacent to the first body part 111, and the first edge part 112 is disposed along a circumferential direction of the first body part 111. For example, the first edge part 112 encircles the circumferential direction of the first body part 111. The second film layer 12 includes a second body part 121 and a second edge part 122, the second edge part 122 is adjacent to the second body part 121, and the second edge part 122 is disposed along a circumferential direction of the second body part 121. For example, the second edge part 122 encircles the circumferential direction of the second body part 121.

As shown in FIG. 11, the first body part 111 and the second body part 121 enclose a first cavity 101. As described in the foregoing embodiments, the first cavity 101 may be in communication with the air pump 30, so that the air pump 30 may inflate the first cavity 101. In this way, after the first cavity 101 is pressurized and expanded, the first film layer 11 presses radial artery blood vessels and ulnar artery blood vessels of a wrist of a human body, thereby facilitating blood pressure measurement.

In some embodiments of a related technology, a blood pressure measurement apparatus 1000 has a plurality of airbags 10. In other words, the blood pressure measurement apparatus 1000 includes a plurality of the first cavities 101. As a result, the blood pressure measurement apparatus 1000 is large in size and heavy in weight, resulting in poor wearing comfort.

In some other embodiments of the related technology, the blood pressure measurement apparatus 1000 has one airbag 10, and a structure of the airbag 10 is simple. The first edge part 112 and the second edge part 122 jointly form a press edge, and the press edge is located outside the airbag 10. During actual use, the press edge may contact and rub against skin of the wrist, thereby degrading wearing experience of a user.

In view of this, as shown in FIG. 11, the first edge part 112 and the second edge part 122 are connected and disposed in a stacked manner, and the first edge part 112 and the second edge part 122 jointly form a first connection part 151. For example, the first edge part 112 and the second edge part 122 may form the first connection part 151 by using a pressing process, and the first film layer 11 and the second film layer 12 may be connected through the first edge part 112 and the second edge part 122 that are pressed together. The first connection part 151 may encircle a circumferential direction of the airbag, or the first connection part 151 may be disposed in a partial circumferential direction of the airbag.

In the airbag 10 provided in embodiments of this application, the first connection part 151 is located in the first cavity 101. According to the foregoing configuration, during actual use, contact friction between at least the first connection part 151 and the skin of the wrist can be avoided, and irritation to the skin of the wrist can be reduced, thereby improving wearing comfort of the user.

In addition, because the first connection part 151 is located in the first cavity 101, areas of the first body part 111 and the second body part 121 can be increased on a basis of an unchanged size of the airbag 10, so that a volume of the first cavity 101 enclosed by the first body part 111 and the second body part 121 can be increased. This helps increase an inflation amount of the airbag 10 and improve measurement accuracy of the blood pressure measurement apparatus 1000. On the contrary, according to the foregoing configuration, the size of the airbag can be reduced on a basis of same measurement accuracy of the blood pressure measurement apparatus. For example, when the airbag 10 has a same width, compared with an effective inflation area in embodiments of the related technology, an effective inflation area in embodiments of this application is increased by twice a width of the first connection part 151.

In some embodiments, as shown in FIG. 10, the first edge part 112 and the second edge part 122 further jointly form a second connection part 12, the second connection part 152 and the first connection part 151 are connected end-to-end in a ring shape and jointly form a connection part 15, and the second connection part 152 is located outside the first cavity 101. For example, in the first film layer 11 and the second film layer 12, a portion of the first edge part 112 and a portion of the second edge part 122 are located in the first cavity 101, and jointly form the first connection part 151. The other portion of the first edge part 112 and the other portion of the second edge part 122 are located outside the first cavity 101, and jointly form the second connection part 152. The first edge part 112 and the second edge part 122 may be connected together through pressing.

Still refer to FIG. 10. The connection part 15 may include a first portion 1501, a second portion 1502, a third portion 1503, and a fourth portion 1504. The first portion 1501 and the second portion 1502 are disposed opposite to each other, the third portion 1503 and the fourth portion 1504 are disposed opposite to each other, and the third portion 1503 and the fourth portion 1504 are connected between the first portion 1501 and the second portion 1502. The first connection part 151 includes the first portion 1501, the second portion 1502, and the third portion 1503, and the second connection part 152 includes the fourth portion 1504. According to the foregoing configuration, a length of the first connection part 151 can be further increased, so that contact friction between the first connection part 151 and the skin of the wrist can be avoided, and irritation to the skin of the wrist can be reduced, thereby improving wearing comfort of the user.

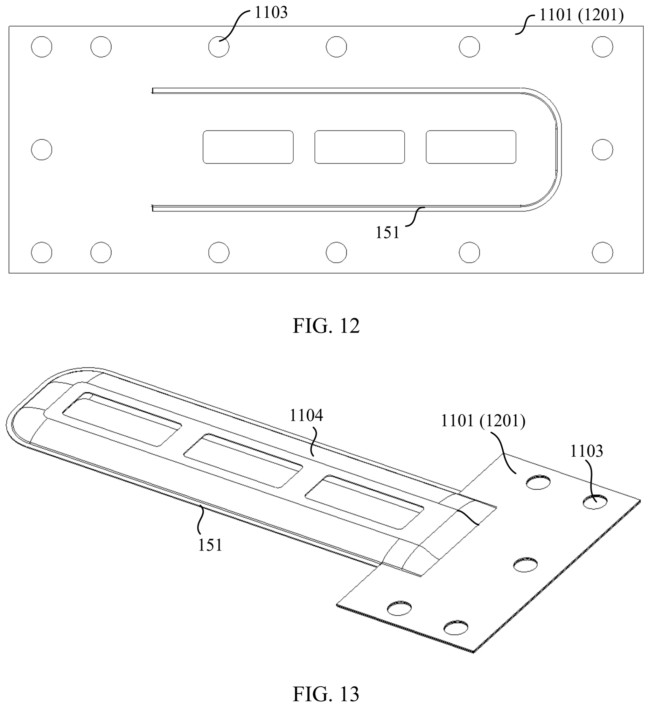

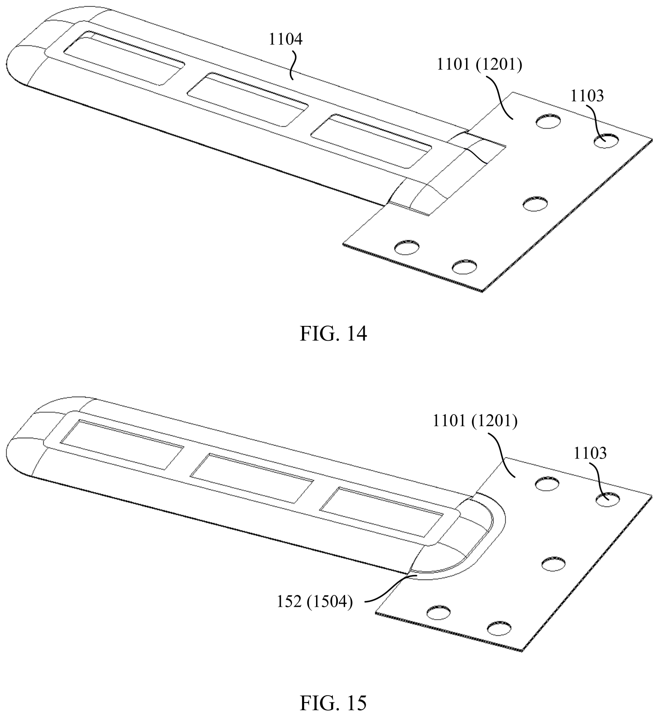

FIG. 12 is a diagram of a structure of a first film material 1101 and a second film material 1201 according to an embodiment of this application. FIG. 13 is a diagram of a structure of a first intermediate body 1104 according to an embodiment of this application. FIG. 14 is a diagram of a structure of a first intermediate body 1104 that is flipped according to an embodiment of this application. FIG. 15 is a diagram of a structure of a formed second connection part 152 according to an embodiment of this application.

The following briefly describes a manufacturing process of the airbag 10. As shown in FIG. 12, the first film material 1101 and the second film material 1201 that are disposed in a stacked manner are provided. The first film material 1101 and the second film material 1201 are pressed together in a specific shape to form the first connection part 151. A positioning hole 1103 is provided on the first film material 1101 and the second film material 1201, and the positioning hole 1103 is configured to perform positioning in a pressing process. In the first film material 1101, a structure that is pressed together with the second film material 1201 is a portion of the first edge part 112. In the second film material 1201, a structure that is pressed together with the first film material 1101 is a portion of the second edge part 122. For example, the first connection part 151 is approximately "U"-shaped, a pressed structure located at a right end of a position shown in the figure is a third portion 1503 of the first connection part 151, a pressed structure located at an upper end of the position shown in the figure is a first portion 1501 of the first connection part 151, and a pressed structure located at a lower end of the position shown in the figure is a second portion 1502 of the first connection part 151.

As shown in FIG. 13, after the first connection part 151 is formed, the first film material 1101 and the second film material 1201 that are outside the first connection part 151 are cut for the first time, to form the first intermediate body 1104. The first connection part 151 is located outside the first intermediate body 1104.

As shown in FIG. 14, after the first film material 1101 and the second film material 1201 are cut for the first time, the first intermediate body 1104 is flipped and shaped. With reference to FIG. 11, it can be learned that, after the first intermediate body 1104 is flipped, the first connection part 151 is located in the first intermediate body 1104.

As shown in FIG. 15, after the first intermediate body 1104 is flipped and shaped, the first film material 1101 and the second film material 1201 are pressed for the second time, to form the second connection part 152. For example, a pressed structure located at a right end of the position shown in the figure is a fourth portion 1504 of the second connection part 152. In the first film material 1101, a structure that is pressed together with the second film material 1201 is a portion of the first edge part 112. In the second film material 1201, a structure that is pressed together with the first film material 1101 is a portion of the second edge part 122. Because the second connection part 152 is adjacent to the first connection part 151, the first connection part 151 and the second connection part 152 jointly form a connection part 15. The first film material 1101 enclosed by the connection part 15 is the first body part 111, the second film material 1201 enclosed by the connection part 15 is the second body part 121, and an enclosed space enclosed by the first body part 111 and the second body part 121 is the first cavity 101.

As shown in FIG. 10, after the first film material 1101 and the second film material 1201 are pressed for the second time, the first film material 1101 and the second film material 1201 that are outside the second connection part 152 are cut for the second time, to form the airbag 10.

In conclusion, the first connection part 151 and the second connection part 152 are disposed (through the foregoing two pressing processes), so that the first film material 1101 and the second film material 1201 can be manufactured to form the first cavity 101. In addition, the first connection part 151 is located in the first cavity 101, so that contact friction between the first connection part 151 and skin of a wrist can be avoided, and irritation to the skin of the wrist can be reduced, thereby improving wearing comfort of a user.

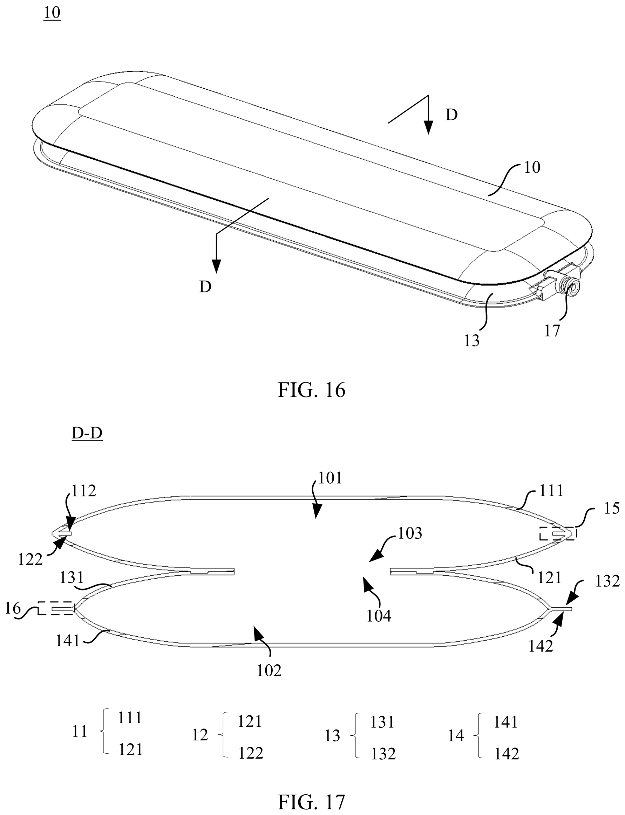

FIG. 16 is a diagram of a structure of another airbag 10 according to an embodiment of this application. FIG. 17 is a cross-sectional view of the airbag 10 in FIG. 16 along a section line D-D.

Still refer to FIG. 16 and FIG. 17. The airbag 10 may include a third film layer 13 and a fourth film layer 14, and the third film layer 13 and the fourth film layer 14 are disposed opposite to each other. For example, shapes of the third film layer 13 and the fourth film layer 14 may be the same. For example, the shapes of the third film layer 13 and the fourth film layer 14 may be approximately round rectangles, so that a shape of the airbag 10 is regular. Certainly, in some other embodiments, the shapes of the third film layer 13 and the fourth film layer 14 may be correspondingly configured based on an actual situation. This is not limited in embodiments of this application.

Still refer to FIG. 17. The third film layer 13 may include a third body part 131 and a third edge part 132, the third edge part 132 is adjacent to the third body part 131, and the third edge part 132 is disposed along a circumferential direction of the third body part 131. For example, the third edge part 132 encircles the circumferential direction of the third body part 131. The fourth film layer 14 may include a fourth body part 141 and a fourth edge part 142, the fourth edge part 142 is adjacent to the fourth body part 141, and the fourth edge part 142 is disposed along a circumferential direction of the fourth body part 141. For example, the fourth edge part 142 encircles the circumferential direction of the fourth body part 141.

The third body part 131 and the fourth body part 141 may enclose a second cavity 102, the third edge part 132 and the fourth edge part 142 are connected and disposed in a stacked manner, and the third body part 131 is connected to the second body part 121. For example, the third film layer 13 is closer to the second film layer 12 than the fourth film layer 14, and the third body part 131 of the third film layer 13 and the second body part 121 of the second film layer 12 may be welded together, so that the airbag 10 can form a double-layer structure. Because the first film layer 11 is configured to be in contact with skin of a wrist, the third film layer 13, the fourth film layer 14, and the second cavity 102 are all located on a side that is of the first cavity 101 and that is away from the skin of the wrist.

With reference to FIG. 17, the third film layer 13 may include a second opening 104, the second film layer 12 may include a first opening 103, and the second opening 104 is in communication with the first opening 103. For example, there may be one or more first openings 103, and a quantity of second openings 104 may be correspondingly set based on the quantity of first openings 103. Similarly, a shape of the second opening 104 may be correspondingly configured based on a shape of the first opening 103. According to the foregoing configuration, air in the second cavity 102 may enter the first cavity 101 sequentially through the second opening 104 and the first opening 103, so that the first cavity 101 is in communication with the second cavity 102.

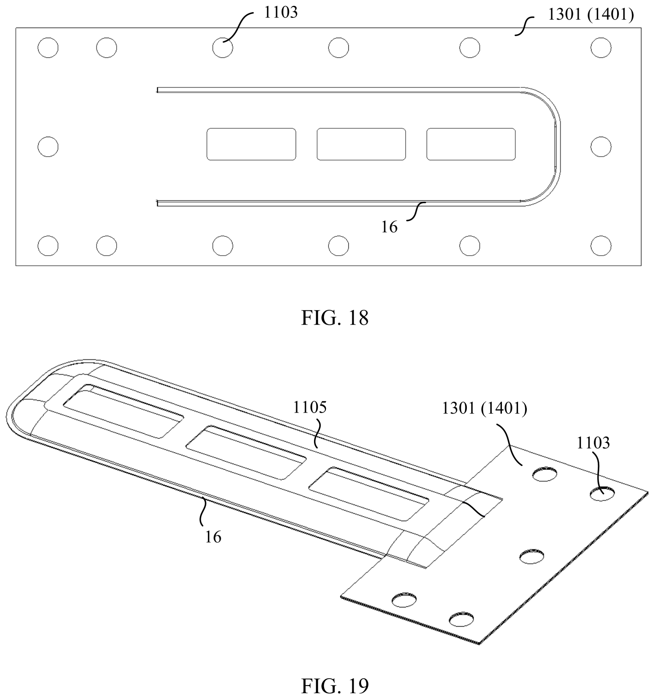

FIG. 18 is a diagram of a structure of a third film material 1301 and a fourth film material 1401 according to an embodiment of this application. FIG. 19 is a diagram of a structure of a second intermediate body 1105 according to an embodiment of this application.

Based on the airbag 10 in the foregoing embodiments, the manufacturing process of the airbag 10 may further include the following steps: As shown in FIG. 18, the third film material 1301 and the fourth film material 1401 that are disposed in a stacked manner are provided, and the third film material 1301 and the fourth film material 1401 are pressed together in a specific shape, to form a portion of a third connection part 16. As shown in FIG. 19, after the portion of the third connection part 16 is formed, the third film material 1301 and the fourth film material 1401 that are outside the portion of the third connection part 16 are cut for the first time, to form the second intermediate body 1105. The portion of the third connection part may be located outside the second intermediate body 1105. Certainly, in some other embodiments, the second intermediate body 1105 may alternatively be flipped, so that at least a portion of the third connection part 16 is located in the second intermediate body 1105.

With reference to FIG. 14, after the first intermediate body 1104 and the second intermediate body 1105 are formed, the second film material 1201 and the fourth film material 1401 may be welded together, so that the first intermediate body 1104 and the second intermediate body 1105 are connected together. After the first intermediate body 1104 and the second intermediate body 1105 are connected together, the first film material 1101, the second film material 1201, the third film material 1301, and the fourth film material 1401 are pressed for the second time, so that the first film material 1101 and the second film material 1201 form the second connection part 152, and the third film material 1301 and the fourth film material 1401 form the other portion of the third connection part 16. After the second pressing, the first film material 1101 and the second film material 1201 that are outside the second connection part 152, as well as the third film material 1301 and the fourth film material 1401 that are outside the other portion of the third connection part 16 are cut for the second time, to form the airbag 10.

Refer to FIG. 16. The airbag 10 may further include an air nozzle 17. The air nozzle 17 is in communication with the second cavity 102, and is connected to both the third film layer 13 and the fourth film layer 14.

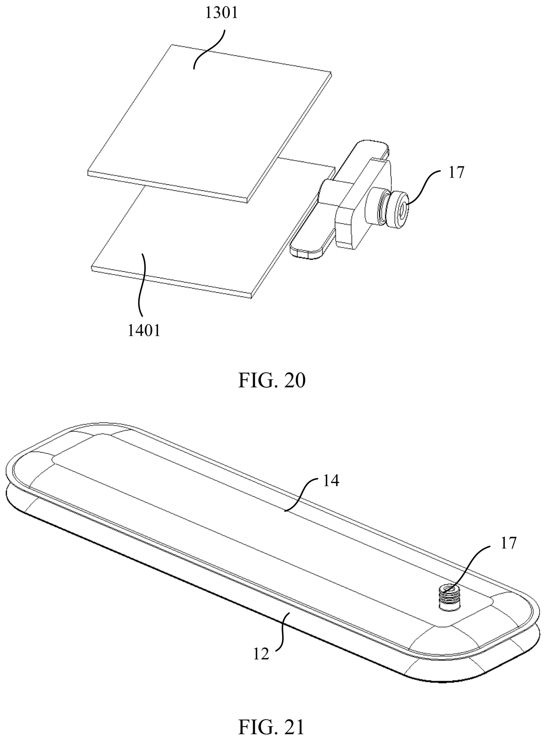

FIG. 20 is a diagram of a structure of an air nozzle according to an embodiment of this application. As shown in FIG. 20, in the manufacturing process of the airbag 10, before the third film material 1301 and the fourth film material 1401 are pressed for the second time, the air nozzle 17 may be placed between the third film material 1301 and the fourth film material 1401, and then the third film material 1301 and the fourth film material 1401 are pressed for the second time, so that the air nozzle 17 is connected to both the third film layer 13 and the fourth film layer 14. According to the foregoing configuration, the airbag 10 and the air nozzle 17 can be integrally formed, thereby simplifying the manufacturing process of the airbag 10 and improving manufacturing efficiency.

Further, an end of the air nozzle 17 is in communication with an air pump 30. The other end of the air nozzle 17 is in communication with the second cavity 102. In conclusion, the air pump 30 can inflate the second cavity 102 through the air nozzle 17, and air in the second cavity 102 enters the first cavity 101, so that the first film layer 11 of the first cavity 101 presses radial artery blood vessels and ulnar artery blood vessels of a wrist of a human body, thereby facilitating blood pressure measurement.

Certainly, in some other embodiments, the air nozzle 17 may alternatively be welded between the third film layer 13 and the fourth film layer 14, or the air nozzle 17 may alternatively be bonded between the third film layer 13 and the fourth film layer 14.

FIG. 21 is a diagram of a structure of another air nozzle 17 according to an embodiment of this application. As shown in FIG. 21, in some other embodiments, the air nozzle 17 may alternatively be located on a side of the airbag 10. For example, the air nozzle 17 may be connected to the fourth film layer 14, and the air nozzle 17 is located on a side that is of the fourth film layer 14 and that is away from the wrist. However, in embodiments of this application, the air nozzle 17 is connected to both the third film layer 13 and the fourth film layer 14, so that the air nozzle 17 can be located at an edge of the airbag 10. This helps reduce an occupied volume of the airbag 10, and helps implement miniaturization of the blood pressure measurement apparatus 1000.

In conclusion, the second cavity 102 is disposed, so that the airbag 10 can be disposed in a double-layer manner. On a premise of an unchanged overall inflation volume of the airbag 10, a width of the airbag 10 can be narrowed, so that a width of a watch strap 100 on which the airbag 10 is mounted is narrowed, thereby improving wearing comfort of the blood pressure measurement apparatus 1000.

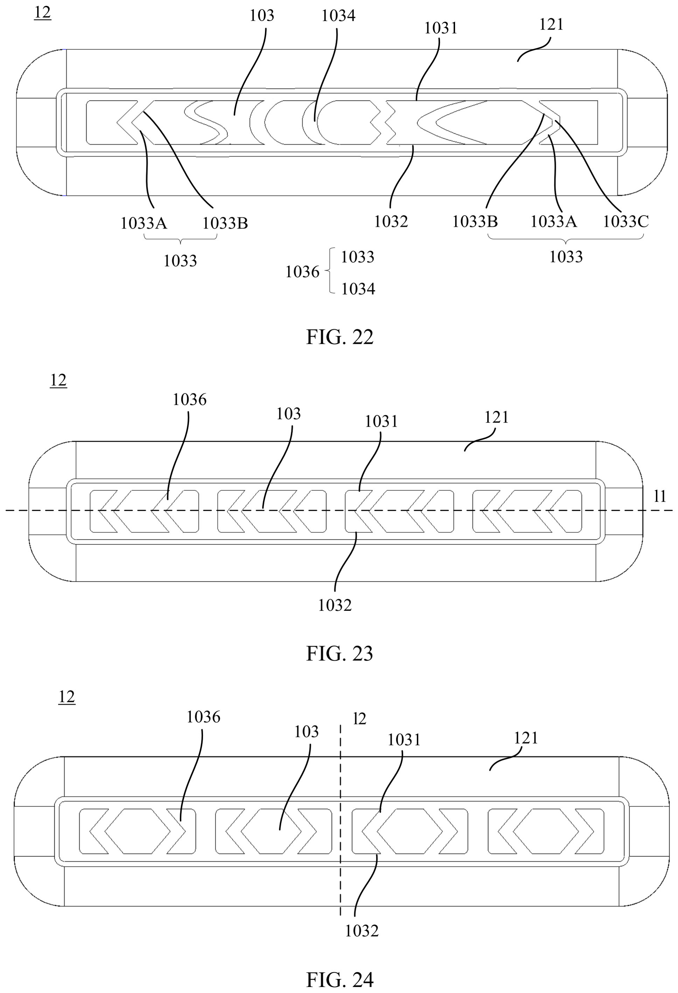

FIG. 22 is a diagram of a structure of a second film layer 12 according to an embodiment of this application.

Refer to FIG. 22. The airbag 10 may further include a first connection film 1036. The first opening 103 includes a first edge 1031 and a second edge 1032 that are disposed opposite to each other, the first connection film 1036 is connected between the first edge 1031 and the second edge 1032, and a plurality of the first connection films 1036 are spaced apart. For example, in the second body part 121, a shape of the first opening 103 may be approximately rectangular. The first edge 1031 and the second edge 1032 of the first opening 103 are disposed in parallel, and extension directions of the first edge 1031 and the second edge 1032 are the same as an extension direction of the airbag 10. As described in the foregoing embodiments, the air in the second cavity 102 may enter the first cavity 101 through the first opening 103, and stability of the first cavity 101 in an expansion process is reduced, further leading to reduced measurement accuracy of the blood pressure measurement apparatus 1000. In embodiments of this application, in the expansion process of the first cavity 101, the plurality of the first connection films 1036 are disposed, so that a limiting effect can be achieved on the first edge 1031 and the second edge 1032 of the first opening 103. This helps reduce a deformation speed of the second film layer 12 in which the first opening 103 is located, thereby improving stability of the first cavity 101 and measurement accuracy of the blood pressure measurement apparatus 1000.

Still refer to FIG. 22. In some embodiments, the first connection film 1036 may include a bent part 1033. For example, the first connection film 1036 may include one or more bent parts 1033. The bent part 1033 may include a first bent edge 1033A and a second bent edge 1033B. The first bent edge 1033A is adjacent to the second bent edge 1033B, and there is a specific included angle. Alternatively, the bent part 1033 may include a third bent edge 1033C. The third bent edge 1033C is connected between the first bent edge 1033A and the second bent edge 1033B. There is a specific included angle between the first bent edge 1033A and the third bent edge 1033C, and there is also a specific included angle between the second bent edge 1033B and the third bent edge 1033C. Certainly, the bent part 1033 in embodiments of this application is not limited to the foregoing two implementations. This is not limited in embodiments of this application.

Still refer to FIG. 22. In some embodiments, the first connection film 1036 may include an arc-shaped part 1034. For example, the first connection film 1036 may include one or more arc-shaped parts 1034. A radius of an arc in which the arc-shaped part 1034 is located, and a center position of the arc in which the arc-shaped part 1034 is located are not limited in embodiments of this application.

Certainly, in some other embodiments, the first connection film 1036 may include both the bent part 1033 and the arc-shaped part 1034. This is not limited in embodiments of this application.

In conclusion, in the expansion process of the first cavity 101, the bent part 1033 and/or the arc-shaped part 1034 are disposed in the first connection film 1036, so that the first connection film 1036 can have a specific stretching amount (herein, the stretching amount is a length that the first connection film 1036 can stretch in a direction perpendicular to the first edge 1031), so that the second body part 121 in which the first opening 103 is located has a specific deformation amount. This helps increase an expansion height and an inflation volume of the first cavity 101 enclosed by the second body part 121 and the first body part 111, thereby improving measurement accuracy of the blood pressure measurement apparatus 1000.

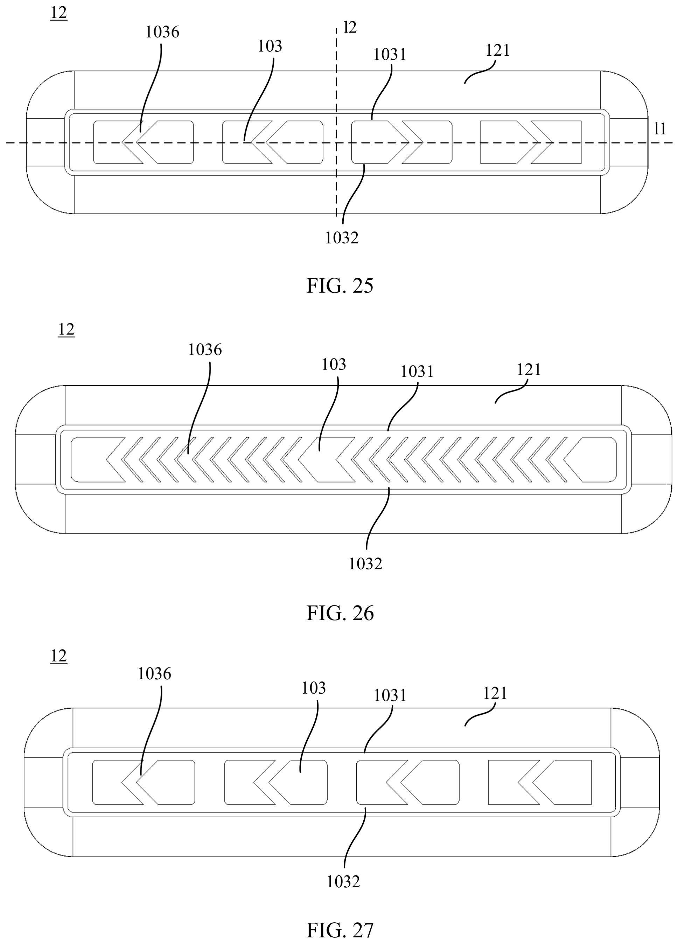

FIG. 23 is a diagram of a structure of another second film layer 12 according to an embodiment of this application. FIG. 24 is a diagram of a structure of another second film layer 12 according to an embodiment of this application. FIG. 25 is a diagram of a structure of another second film layer 12 according to an embodiment of this application. FIG. 26 is a diagram of a structure of another second film layer 12 according to an embodiment of this application. FIG. 27 is a diagram of a structure of another second film layer 12 according to an embodiment of this application.

In some embodiments, an arrangement manner of the plurality of the first connection films 1036 may be adjusted according to an actual application requirement. For example, as shown in FIG. 23, the plurality of the first connection films 1036 may be disposed symmetric with respect to a center line 11 of the first opening 103. Herein, the center line of the first opening 103 may be perpendicular to the first edge 1031. Alternatively, as shown in FIG. 24, the plurality of the first connection films 1036 may be disposed symmetric with respect to a center line l2 of the first opening 103, and the center line of the first opening 103 may be parallel to the first edge 1031. Alternatively, as shown in FIG. 25, the plurality of the first connection films 1036 may be disposed symmetric with respect to a center line 11 of the first opening 103, and disposed symmetric with respect to a center line l2 of the first opening 103. According to the foregoing configuration, the first edge 1031 and the second edge 1032 of the first opening 103 are evenly subject to a force. This avoids stress concentration on the second film layer 12 in which the first opening 103 is located, thereby avoiding damage to the second film layer 12, and helping prolong life of use of the airbag 10.

In some other embodiments, as shown in FIG. 26, shapes of the plurality of the first connection films 1036 may be the same, and the plurality of the first connection films 1036 are arranged in an array in a direction parallel to the first edge 1031. Alternatively, as shown in FIG. 27, a portion of the plurality of the first connection films 1036 may include the bent part 1033 and/or the arc-shaped part 1034, and the other portion of the plurality of the first connection films 1036 may not include the bent part 1033 and/or the arc-shaped part 1034.

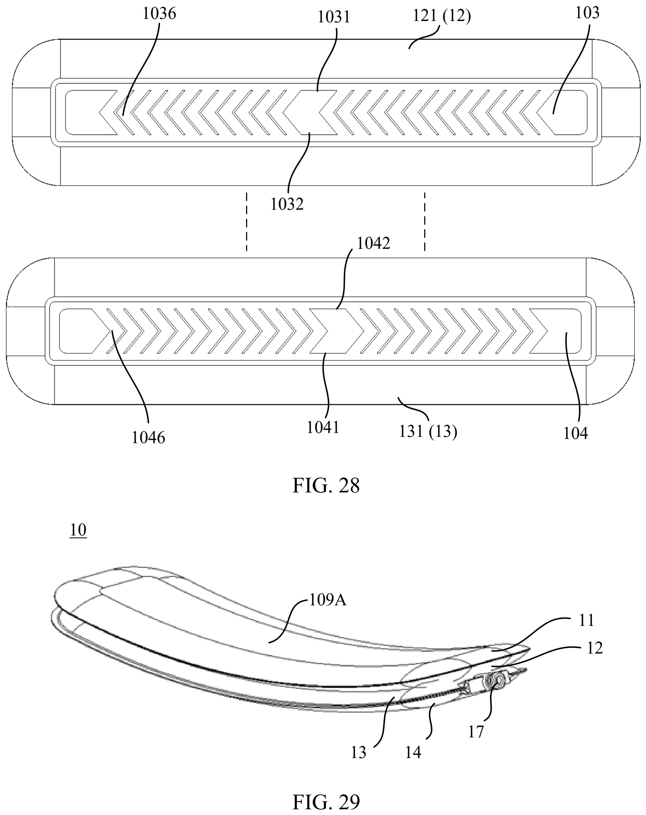

FIG. 28 is a diagram of structures of a second film layer 12 and a third film layer 13 according to an embodiment of this application.

Still refer to FIG. 28. A second connection film 1046 may be further included. The second opening 104 includes a third edge 1041 and a fourth edge 1042 that are disposed opposite to each other, the second connection film 1046 is connected between the third edge 1041 and the fourth edge 1042, and a plurality of the second connection films 1046 are spaced apart. For example, in the third body part 131, a shape of the second opening 104 may be approximately rectangular. The third edge 1041 and the fourth edge 1042 of the second opening 104 are disposed in parallel, and extension directions of the third edge 1041 and the fourth edge 1042 are the same as an extension direction of the airbag 10. As described in the foregoing embodiments, in the expansion process of the second cavity 102, the plurality of the second connection films 1046 are disposed, so that a limiting effect can be achieved on the third edge 1041 and the fourth edge 1042 of the second opening 104. This helps reduce a deformation speed of the third film layer 13 in which the second opening 104 is located, thereby improving stability of the second cavity 102, stability of the first cavity 101, and measurement accuracy of the blood pressure measurement apparatus 1000.

Further, the plurality of the second connection films 1046 may be disposed symmetric to the plurality of the first connection films 1036. For example, the plurality of the second connection films 1046 are disposed symmetric to the plurality of the first connection films 1036, so that a communication area between the first opening 103 and the second opening 104 can be increased, and a ventilation volume between the first cavity 101 and the second cavity 102 can be increased. This can increase an expansion height and an inflation volume of the first cavity 101, thereby improving measurement accuracy of the blood pressure measurement apparatus 1000.

FIG. 29 is a diagram of a structure of another airbag 10 according to an embodiment of this application. As shown in FIG. 29, a difference from the structure of the airbag 10 in the foregoing embodiments lies in that, in this embodiment, the first film layer 11, the second film layer 12, the third film layer 13, and the fourth film layer 14 all include an arc-shaped surface 109A. For example, radians of the arc-shaped surfaces 109A in the first film layer 11, the second film layer 12, the third film layer 13, and the fourth film layer 14 are the same.

It may be understood that, during use of the airbag 10, because the airbag 10 needs to be attached to the wrist, the airbag 10 is prone to wrinkles. Consequently, signal fluctuation occurs during data collection, and measurement accuracy of the blood pressure measurement apparatus 1000 is affected. According to the foregoing configuration, the airbag 10 is easily attached to the wrist, thereby avoiding the wrinkles during use of the airbag 10, and improving measurement accuracy of the blood pressure measurement apparatus 1000.

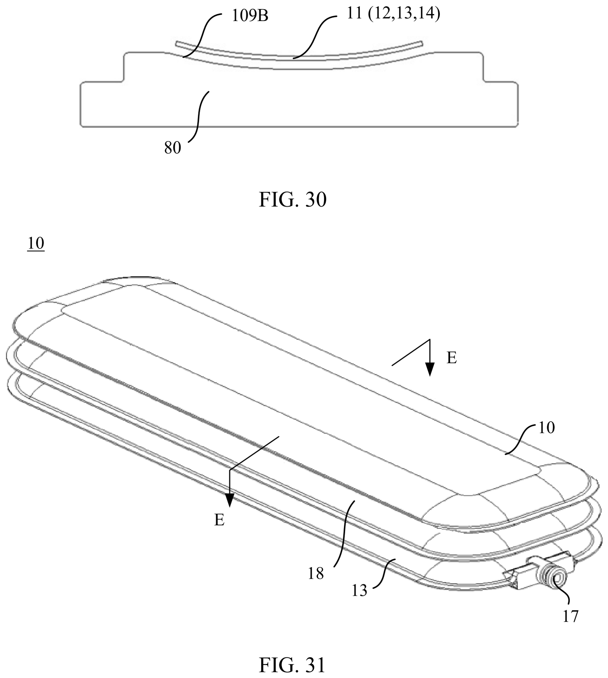

In some embodiments, after a film material is stretched, a corresponding mold may be used to press the film material. FIG. 30 is a diagram of a structure of a mold 80 according to an embodiment of this application. As shown in FIG. 30, the mold 80 may include an arc-shaped forming surface 109B. The first film layer 11, the second film layer 12, the third film layer 13, and the fourth film layer 14 that have the arc-shaped surface 109A can be formed through the foregoing preparation process.

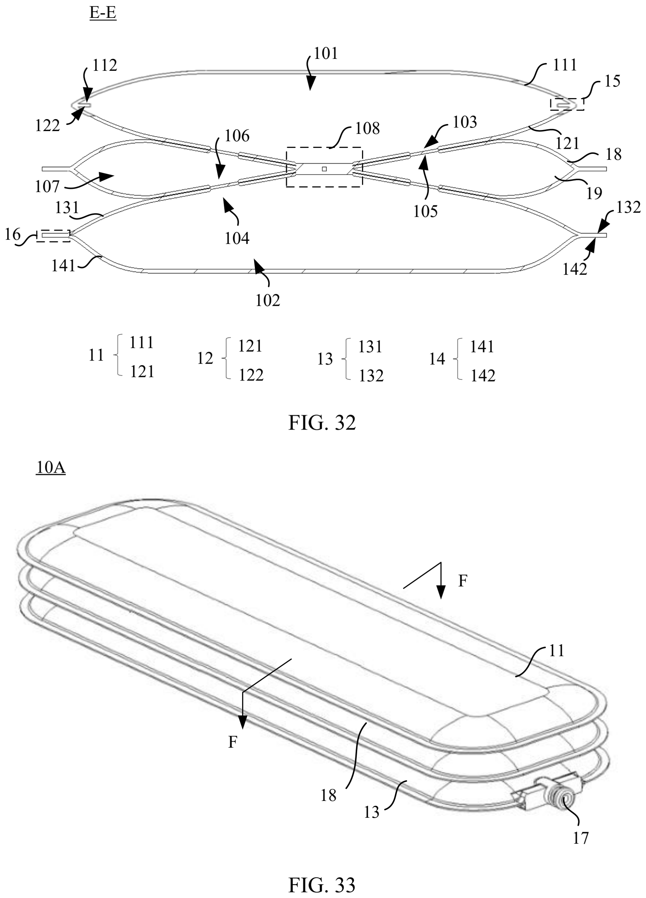

FIG. 31 is a diagram of a structure of another airbag 10 according to an embodiment of this application. FIG. 32 is a cross-sectional view of the airbag 10 in FIG. 31 along a section line E-E.

Refer to FIG. 31 and FIG. 32. The airbag 10 may further include a first intermediate film layer 18 and a second intermediate film layer 19, the first intermediate film layer 18 and the second intermediate film layer 19 enclose an intermediate cavity 107, and the intermediate cavity 107 is located between the first cavity 101 and the second cavity 102. For example, the first intermediate film layer 18 and the second intermediate film layer 19 may be located between the second film layer 12 and the third film layer 13, and the first intermediate film layer 18 may be closer to the second film layer 12 than the second intermediate film layer 19.

Still refer to FIG. 31 and FIG. 32. The airbag 10 may further include a bonding part 108, the bonding part 108 is configured to connect the second film layer 12, the first intermediate film layer 18, the second intermediate film layer 19, and the third film layer 13, the bonding part 108 is located between the first cavity 101 and the second cavity 102, and the intermediate cavity 107 is disposed along a circumferential direction of the bonding part 108. For example, the bonding part 108 may have a specific extension length along an extension direction of the airbag 10, and the intermediate cavity 107 may be an annular enclosed space. Based on this, the second cavity 102 is in communication with the first cavity 101 through the intermediate cavity 107. In an actual application process, an air pump 30 may enter the second cavity 102 through an air nozzle 17. After air in the second cavity 102 enters the intermediate cavity 107, air in the intermediate cavity 107 may enter the first cavity 101, so that the first film layer 11 of the first cavity 101 presses radial artery blood vessels and ulnar artery blood vessels of a wrist of a human body, thereby facilitating blood pressure measurement.

According to the foregoing configuration, in an inflation process of the airbag 10, because a volume of the intermediate cavity 107 expands, the intermediate cavity 107 squeezes the second film layer 12 in contact with the intermediate cavity 107, so that an edge position of the second body part 121 of the second film layer 12 moves toward a direction close to the wrist. This increases an effective pressing area between the first film layer 11 and the wrist, thereby improving measurement accuracy of the blood pressure measurement apparatus 1000. Herein, the effective pressing area is an area in which the first film layer 11 can press the radial artery blood vessels and the ulnar artery blood vessels of the wrist of the human body to enable the blood pressure measurement apparatus 1000 to obtain the pulse wave signal.

In addition, because the intermediate cavity 107 is disposed in the circumferential direction of the bonding part 108, the intermediate cavity 107 can stabilize the first cavity 101. This helps prevent the first cavity 101 from deviating toward one side, thereby improving stability of the first cavity 101 and measurement accuracy of the blood pressure measurement apparatus 1000.

Further, the intermediate cavity 107 and the second cavity 102 are disposed, so that the airbag 10 can be of a three-layer structure. On a premise of an unchanged overall inflation volume of the airbag 10, a width of the airbag 10 can be further narrowed, so that a width of a watch strap 100 on which the airbag 10 is mounted is further narrowed, thereby improving wearing comfort of the blood pressure measurement apparatus 1000.

Still refer to FIG. 32. The first intermediate film layer 18 may include a first intermediate opening 105, the first intermediate opening 105 is in communication with the first opening 103, the second intermediate film layer 19 may include a second intermediate opening 106, and the second intermediate opening 106 is in communication with the second opening 104. For example, the air in the second cavity 102 may enter the intermediate cavity 107 sequentially through the second opening 104 and the second intermediate opening 106, and the air in the intermediate cavity 107 may enter the first cavity 101 sequentially through the first intermediate opening 105 and the first opening 103. The first intermediate opening 105 may be correspondingly disposed based on a quantity and shapes of the first openings 103, and the second intermediate opening 106 may be correspondingly disposed based on a quantity and shapes of the second openings 104. According to the foregoing configuration, the first cavity 101 can be in communication with the second cavity 102 through the intermediate cavity 107.

Further, the first connection film 1036 in the foregoing embodiments may be disposed in the first intermediate opening 105 and the second intermediate opening 106, to increase an expansion height and an inflation volume of the intermediate cavity 107, thereby further improving measurement accuracy of the blood pressure measurement apparatus 1000.

Still refer to FIG. 32. The second film layer 12, the first intermediate film layer 18, the second intermediate film layer 19, and the third film layer 13 may jointly form the bonding part 108. For example, the second film layer 12, the first intermediate film layer 18, the second intermediate film layer 19, and the third film layer 13 may be sequentially disposed in a stacked manner and pressed together to form the bonding part 108. According to the foregoing configuration, connection strength between the second film layer 12, the first intermediate film layer 18, the second intermediate film layer 19, and the third film layer 13 can be improved. Further, the second film layer 12 and the first intermediate film layer 18, and the second intermediate film layer 19 and the third film layer 13 may be further connected in a sealed manner, to further improve connection reliability between adjacent layers of the airbag 10.

Certainly, in some other embodiments, the bonding part 108 may further include a bonding layer, and two adjacent layers among the second film layer 12, the first intermediate film layer 18, the second intermediate film layer 19, and the third film layer 13 may be bonded together through the bonding layer. Alternatively, the bonding part 108 may further include a welding layer, and two adjacent layers among the second film layer 12, the first intermediate film layer 18, the second intermediate film layer 19, and the third film layer 13 may be welded together through the welding layer.

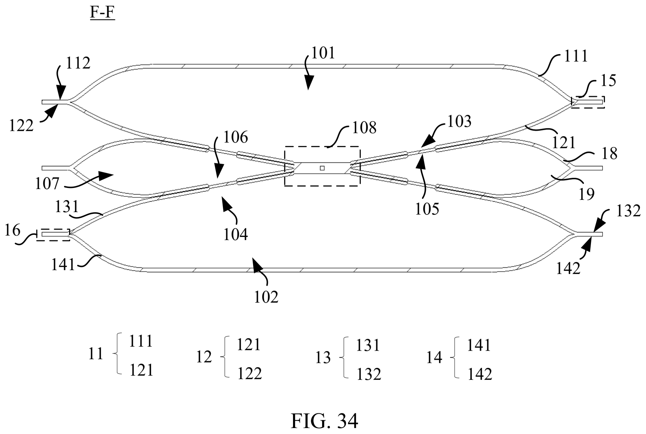

FIG. 33 is a diagram of a structure of another airbag 10A according to an embodiment of this application. FIG. 34 is a cross-sectional view of the airbag in FIG. 33 along a section line F-F.

Refer to FIG. 33 and FIG. 34. An embodiment of this application further provides an airbag 10A. The airbag 10A includes a first film layer 11 and a second film layer 12, the first film layer 11 and the second film layer 12 are disposed opposite to each other, and the first film layer 11 is configured to be in contact with skin. The first film layer 11 includes a first body part 111 and a first edge part 112, the first edge part 112 is adjacent to the first body part 111, and the first edge part 112 is disposed along a circumferential direction of the first body part 111. The first body part 111 and the second body part 121 enclose a first cavity 101. For structures of the first body part 111, the first edge part 112, the second body part 121, and the second edge part 122, refer to the descriptions in the foregoing embodiments. Details are not described herein again.

The first edge part 112 and the second edge part 122 are connected and disposed in a stacked manner, and the first edge part 112 and the second edge part 122 jointly form a connection part 15. It can be learned from FIG. 32 that, a difference from the structure in FIG. 32 lies in that the connection part 15 is located outside the first cavity 101. For example, the connection part 15 may encircle a circumferential direction of the first cavity 101.

The airbag 10A further includes a third film layer 13 and a fourth film layer 14, and the third film layer 13 and the fourth film layer 14 are disposed opposite to each other. The third film layer 13 may include a third body part 131 and a third edge part 132, the third edge part 132 is adjacent to the third body part 131, and the third edge part 132 is disposed along a circumferential direction of the third body part 131. The fourth film layer 14 may include a fourth body part 141 and a fourth edge part 142, the fourth edge part 142 is adjacent to the fourth body part 141, and the fourth edge part 142 is disposed along a circumferential direction of the fourth body part 141. The third body part 131 and the fourth body part 141 may enclose a second cavity 102. For structures of the third body part 131, the third edge part 132, the fourth body part 141, and the fourth edge part 142, refer to the descriptions in the foregoing embodiments. Details are not described herein again.

The third edge part 132 and the fourth edge part 142 are connected and disposed in a stacked manner, the third edge part 132 and the third edge part 132 jointly form a third connection part 16, and the third connection part 16 is located outside the second cavity 102. For example, the third connection part 16 may encircle a circumferential direction of the second cavity 102.

The airbag 10A further includes a first intermediate film layer 18 and a second intermediate film layer 19, the first intermediate film layer 18 and the second intermediate film layer 19 enclose an intermediate cavity 107, and the intermediate cavity 107 is located between the first cavity 101 and the second cavity 102. The airbag 10A further includes a bonding part 108, the bonding part 108 is configured to connect the second film layer 12, the first intermediate film layer 18, the second intermediate film layer 19, and the third film layer 13, the bonding part 108 is located between the first cavity 101 and the second cavity 102, and the intermediate cavity 107 is disposed along a circumferential direction of the bonding part 108. For structures of the first intermediate film layer 18 and the second intermediate film layer 19, refer to the descriptions in the foregoing embodiments. Details are not described herein again.

In an actual application process, an air pump 30 may enter the second cavity 102 through an air nozzle 17. After air in the second cavity 102 enters the intermediate cavity 107, air in the intermediate cavity 107 may enter the first cavity 101, so that the first film layer 11 of the first cavity 101 presses radial artery blood vessels and ulnar artery blood vessels of a wrist of a human body, thereby facilitating blood pressure measurement.

As described in the foregoing embodiments, according to the airbag 10A provided in this embodiment of this application, an effective pressing area between the first film layer 11 and the wrist is increased. This helps improve measurement accuracy of a blood pressure measurement apparatus 1000. In addition, the intermediate cavity 107 can stabilize the first cavity 101. This helps improve stability of the first cavity 101 and measurement accuracy of the blood pressure measurement apparatus 1000. Further, the intermediate cavity 107 and the second cavity 102 are disposed, so that a width of the airbag 10A can be further narrowed, and a width of a watch strap 100 on which the airbag 10A is mounted is further narrowed, thereby improving wearing comfort of the blood pressure measurement apparatus 1000.

The foregoing descriptions are merely specific implementations of this application, but are not intended to limit the protection scope of this application. Any variation or replacement readily figured out by a person skilled in the art within the technical scope disclosed in this application shall fall within the protection scope of this application. Therefore, the protection scope of this application shall be subject to the protection scope of the claims.

## Claims

1. An airbag for blood pressure measurement, comprising a first film layer and a second film layer, wherein the first film layer and the second film layer are disposed opposite to each other, and the first film layer is configured to be in contact with skin;
the first film layer comprises a first body part and a first edge part, the first edge part is adjacent to the first body part, and the first edge part is disposed along a circumferential direction of the first body part;
the second film layer comprises a second body part and a second edge part, the second edge part is adjacent to the second body part, and the second edge part is disposed along a circumferential direction of the second body part;
the first body part and the second body part enclose a first cavity, the first edge part and the second edge part are connected and disposed in a stacked manner, and the first edge part and the second edge part jointly form a first connection part; and
the first connection part is located in the first cavity.

2. The airbag according to claim 1, wherein the first edge part and the second edge part further jointly form a second connection part, the second connection part and the first connection part are connected end-to-end in a ring shape and jointly form a connection part, and the second connection part is located outside the first cavity.

3. The airbag according to claim 2, wherein the connection part comprises a first portion, a second portion, a third portion, and a fourth portion, the first portion and the second portion are disposed opposite to each other, the third portion and the fourth portion are disposed opposite to each other, and the third portion and the fourth portion are connected between the first portion and the second portion; and
the first connection part comprises the first portion, the second portion, and the third portion, and the second connection part comprises the fourth portion.

4. The airbag according to any one of claims 1 to 3, wherein the airbag comprises a third film layer and a fourth film layer, and the third film layer and the fourth film layer are disposed opposite to each other;
the third film layer comprises a third body part and a third edge part, the third edge part is adjacent to the third body part, and the third edge part is disposed along a circumferential direction of the third body part;
the fourth film layer comprises a fourth body part and a fourth edge part, the fourth edge part is adjacent to the fourth body part, and the fourth edge part is disposed along a circumferential direction of the fourth body part;
the third body part and the fourth body part enclose a second cavity, the third edge part and the fourth edge part are connected and disposed in a stacked manner, and the third body part is connected to the second body part; and
the third body part comprises a second opening, the second body part comprises a first opening, and the second opening is in communication with the first opening.

5. The airbag according to claim 4, wherein the airbag further comprises an air nozzle, and the air nozzle is in communication with the second cavity and is connected between the third edge part and the fourth edge part.

6. The airbag according to claim 4 or 5, further comprising a first connection film, wherein the first opening comprises a first edge and a second edge that are disposed opposite to each other, the first connection film is connected between the first edge and the second edge, and a plurality of the first connection films are spaced apart.

7. The airbag according to claim 6, wherein the first connection film comprises a bent part, and/or the first connection film comprises an arc-shaped part.

8. The airbag according to claim 7, further comprising a second connection film, wherein the second opening comprises a third edge and a fourth edge that are disposed opposite to each other, the second connection film is connected between the third edge and the fourth edge, a plurality of the second connection films are spaced apart, and the plurality of the second connection films are disposed symmetric to the plurality of the first connection films.

9. The airbag according to any one of claims 1 to 8, wherein the airbag further comprises a first intermediate film layer and a second intermediate film layer, the first intermediate film layer and the second intermediate film layer enclose an intermediate cavity, and the intermediate cavity is located between the first cavity and the second cavity; and
the airbag further comprises a bonding part, the bonding part is configured to connect the second film layer, the first intermediate film layer, the second intermediate film layer, and the third film layer, the bonding part is located between the first cavity and the second cavity, the intermediate cavity is disposed along a circumferential direction of the bonding part, and the second cavity is in communication with the first cavity through the intermediate cavity.

10. The airbag according to claim 9, wherein the first intermediate film layer comprises a first intermediate opening, the first intermediate opening is in communication with the first opening, the second intermediate film layer comprises a second intermediate opening, and the second intermediate opening is in communication with the second opening.

11. The airbag according to claim 9, wherein the second film layer, the first intermediate film layer, the second intermediate film layer, and the third film layer are sequentially stacked to form the bonding part.

12. A blood pressure measurement apparatus, comprising a mounting member, a pipeline, an air pump, and the airbag according to any one of claims 1 to 11, wherein both the pipeline and the airbag are located in the mounting member, one end of the pipeline is in communication with the air pump, and the other end of the pipeline is in communication with the airbag.

13. The blood pressure measurement apparatus according to claim 12, wherein the pipeline comprises a first pipe section, a telescopic member, and a second pipe section, the telescopic member is in communication with both the first pipe section and the second pipe section, the first pipe section is connected to the mounting member, and the second pipe section is in communication with the airbag.

14. The blood pressure measurement apparatus according to claim 13, wherein the telescopic member comprises a bellow.

15. A wearable device, comprising the blood pressure measurement apparatus according to any one of claims 12 to 14.

16. The wearable device according to claim 15, further comprising an inflation part, a first valve body, and a second valve body, wherein the inflation part comprises an inflation cavity, an air inlet hole of the inflation cavity is in communication with external air, the first valve body is located in the air inlet hole of the inflation cavity, the first valve body is configured to block or open the air inlet hole of the inflation cavity, an air outlet hole of the inflation cavity is in communication with an airbag, the second valve body is located in the air outlet hole of the inflation cavity, and the second valve body is configured to block or open the air outlet hole of the inflation cavity.

17. The wearable device according to claim 15 or 16, further comprising an air release part and a third valve body, wherein the air release part comprises an air release cavity, an air inlet of the air release cavity is in communication with the airbag, an air outlet of the air release cavity is in communication with the external air, the third valve body is located in the air release cavity, the third valve body is configured to block or open the air outlet of the air release cavity, and the third valve body is further configured to block or open the air inlet of the air release cavity.
